# FASCICULE DE BREVET EUROPEEN

(11) **EP 1 843 804 B1**
(45) Date de publication et mention de la délivrance du brevet: **18.05.2011**
(21) Numéro de dépôt: 06709223.9
(22) Date de dépôt: 02.02.2006
(51) Int. Cl.: A61L 29/14, A61L 31/14

(54) **PERFECTIONNEMENTS AUX EMBOUTS DE PONCTION**
VERBESSERUNGEN VON SPRITZENKANÜLEN
IMPROVEMENS TO PONCTURE TIPS

(30) Priorité: 03.02.2005 FR 0501072
(43) Date de publication de la demande: 17.10.2007
(73) Titulaire: Vygon, 95440 Ecouen (FR)
(72) Inventeur: CARREZ, Jean-Luc, F-95440 Ecouen (FR); DALLE, Valéry, F-60270 Gouvieux (FR)
(74) Mandataire: Texier, Christian
(86) Numéro de dépôt international: PCT/FR2006/000231
(87) Numéro de publication internationale: WO 2006/082314

(56) Documents cités:
- EP-A- 0 529 675
- WO-A-86/03980
- WO-A-95/08296
- WO-A-02/085188
- WO-A-2005/077279
- FR-A- 2 809 625
- GB-A- 2 327 614
- US-A1- 2003 120 308
- PATENT ABSTRACTS OF JAPAN vol. 2000, no. 15, 6 avril 2001 (2001-04-06) & JP 2000 354626 A (TAKEZAWA SHINGO; NIKKISO CO LTD), 26 décembre 2000 (2000-12-26)
- PATENT ABSTRACTS OF JAPAN vol. 2003, no. 11, 5 novembre 2003 (2003-11-05) & JP 2003 205036 A (UNIV NIHON; IR:KK), 22 juillet 2003 (2003-07-22)

## Description

L'invention concerne les embouts utilisés dans le domaine médical pour couper la peau jusqu'à un site, par exemple pour l'introduction d'un cathéter ou d'une sonde dans le site.

On a proposé (FR 2 809 625) de constituer un embout effilé, à l'extrémité distale arrondie d'une aiguille métallique de ponction à usage médical, par une goutte d'un matériau inerte déposée par trempage sur cette extrémité et formant après durcissement une pointe acérée apte à se dissoudre dans les liquides corporels, de telle sorte que le canal de l'aiguille soit ouvert à cette extrémité après dissolution de l'embout pour permettre l'injection d'une substance et que le personnel ne puisse se piquer lors du retrait de l'aiguille après usage et de son élimination.

Cette solution appelle notamment les remarques suivantes :
- la formation d'une pointe effilée par trempage peut être insuffisante pour assurer une ponction correcte ;
- la conception d'un tel embout est incompatible avec un embout qui viendrait de fabrication avec le tube ;
- la dissolution de l'embout dans le corps peut être tout à fait indésirable, notamment dans le cas d'une veine ;
- en cas de dissolution imparfaite de l'embout, le tube peut rester bouché ;
- la dissolution de l'embout est difficile à contrôler avant retrait de l'aiguille.

On a également proposé (publication JP 2000- 354626) de réaliser une aiguille de ponction qui présente un biseau coupant en matériau à mémoire de forme, pour que la section de passage du biseau soit modifiée sous l'effet de la température du corps.

La présente invention a pour but de fournir un embout à biseau coupant, capable de déformation dans le corps jusqu'à devenir atraumatique, dans des conditions contrôlées, sans perte de matière, sans risque d'occlusion du tube qui porte l'embout, et apte à venir de fabrication avec le tube.

Un tel embout est particulièrement destiné à constituer l'extrémité distale d'un tube cathéter, notamment pour l'introduction du cathéter dans une veine ou dans une artère.

Un autre but de l'invention est de fournir un tube muni d'un tel embout et dont la rigidité puisse varier en service, de façon contrôlée, entre un rigidité suffisante pour que le tube avec son embout constitue une aiguille de ponction et une rigidité diminuée en sorte que le tube ait la souplesse d'un tube cathéter.

L'embout de ponction de l'invention est constitué par un tronçon de tube creux en matériau à mémoire de forme qui présente à son extrémité distale un biseau coupant stable et rigide conformé pour découper la peau et pénétrer dans le site, caractérisé en ce que le biseau coupant a la mémoire d'un état où il est non coupant et en ce qu'il est apte à changer d'état pour reprendre cet état atraumatique lorsqu'il est soumis à une condition prévalente dans le site ou sous l'action d'un stimuli commandé de l' extérieur.

Le biseau peut devenir atraumatique en raison d'une perte de rigidité et/ou d'une conformation atraumatique.

De préférence, le changement d'état du biseau est déclenché par la température dans le site ou par une température supérieure créée artificiellement.

Ce mode de déclenchement n'est pas limitatif et d'autres agents de déclenchement du changement de configuration sont par exemple, le pH dans le site, le contact du sang ou d'un autre liquide dans le site, une stimulation électrique commandée depuis l'extérieur du site.

De nombreuses solutions ont déjà été proposées pour fabriquer un tube à usage médical à mémoire de forme et il existe de très nombreuses publications sur ce sujet.

A titre d'exemples, on citera notamment les publications suivantes, qui font elles-mêmes référence à d'autres publications :
- la publication US 5 674 242 décrit des tubes polymères pour dispositif endoprosthétique constitués d'un matériau à mémoire de forme formulé pour avoir une transition d'état activée par un stimuli thermique ;
- la publication US 5 964 744 décrit des compositions polymères médicales ayant une mémoire de forme pour fabriquer un cathéter à implantation aisée dans l'urètre et apte à y adopter une configuration voulue ;
- la publication WO 86/03980 décrit de nombreux exemples de composition polymères à mémoire de forme qui ramollissent à des températures prédéterminées;
- la publication WO 02/085 188 décrit une aiguille métallique à usage médical qui présente une extrémité distale qui a la mémoire d'une configuration courbe et qui reprend cette configuration lorsqu'elle est implantée, ce qui lui permet de contourner un obstacle ;
- la publication WO 99/42 172 décrit une structure implantable comprenant un tube composite capable de se déformer après implantation dans le corps. Ce tube comprend des segments en matériau polymère à mémoire de forme thermosensible. La déformation est conçue pour permettre au tube de s'accommoder de structures myocardiales difficiles ;
- la publication WO 95/08 296 décrit une aiguille métallique pour suture destinée à des opérations endoscopiques : l'aiguille est droite à température normale et a la mémoire d'une forme courbe qu'elle reprend à la température du corps ;
- la publication WO 96/11 721 décrit un cathéter à mémoire de forme qui peut passer d'une configuration rigide à une configuration flexible ;
- la publication US 6 059 815 décrit un actuateur thérapeutique en matériau à base de polyuréthane à mémoire de forme dont la transformation est déclenchée par la température ;
- la publication EP 0 422 693 décrit des cathéters à mémoire de forme qui changent de configuration sous l'effet de la température et des procédés pour fabriquer ces cathéters ;
- la publication EP 0 931 559 décrit un cathéter épidural dont l'extrémité distale a la mémoire d'une forme en J ;
- la publication US 2002/01 42 119 décrit de nombreux exemples de matériaux à mémoire de forme pour la fabrication ;
- la publication FR 2 641 692 décrit un bouchon en matériau polymère à mémoire de forme déformable sous l'effet de la température pour obturer une brèche dans une application médicale.

Ces publications ne donnent que des aperçus sur des utilisations particulières de la mémoire de forme dans le domaine médical et l'homme du métier dispose d'une documentation générale encore beaucoup plus importante sur les matériaux et les procédés utilisables pour mettre en oeuvre la mémoire de forme.

On peut citer à titre d'exemples anciens les publications US 4 053 548 et US 4 193 899 qui décrivent des procédés pour conférer une mémoire de forme thermosensible à des matériaux élastomères particuliers.

Des matériaux variés ont été préconisés comme des alliages de nickel ou de cuivre (Ni-Ti, Cu-Al-Ni, Ni-Al, Mn-Cu, Fe-Ni, Cu-Zn-Al, Ni-Mn-Ga, notamment le Nitinol™, des chlorures de polyvinyle, des copolymères blocs alliés à des copolymères greffés, des compositions élastomères sulfonatées, des polynorbornènes, des copolymères styrène butadiène, des polyuréthanes, des polyéther uréthanes, des polyéthers polyamides, des polycarbonates, des dérives de cellulose, etc.

La présente invention envisage plus particulièrement l'utilisation de matériaux plastique à mémoire de forme, composés de plusieurs polymères qui sont moulés dans une première forme, qui est celle souhaitée lors de l'utilisation, et qu'on contraint ensuite dans une deuxième forme avant utilisation. Celle-ci est imposée par l'un des polymères. Au déclenchement d'un phénomène, le matériau imposant la deuxième forme cède et le produit reprend sa première forme. Le déclencheur peut être la température, des liquides, de la lumière UV...

Pour fabriquer l'embout de l'invention à mémoire de forme thermosensible, on peut entre autres, mettre en oeuvre un procédé dans lequel :
- on moule l'embout dans un matériau polymère présentant une température choisie de transformation de phase, en lui conférant la forme atraumatique souhaitée ;
- on chauffe l'embout à une température supérieure à la température de transformation de phase pour le ramollir et on impose à l'embout ramolli de prendre la forme voulue pour la ponction ;
- on refroidit l'embout jusqu'à température ambiante.

L'embout ainsi préparé est apte à conserver la forme dans laquelle il peut servir d'embout de ponction mais revient dans une veine ou une artère à la forme atraumatique si la température de transformation de phase existe in situ, et conserve sa forme atraumatique au sortir de la veine et du corps.

De préférence, l'embout est venu de moulage à l'extrémité d'un tube dont il est solidaire.

La forme atraumatique du biseau de l'embout peut varier selon les réalisations. Il peut s'agir par exemple d'un arrondissement de l'ouverture de sortie du biseau (au lieu d'être oblongue et en pointe dans la configuration coupante), d'un boulage du biseau de l'embout (au lieu d'être aigüe dans la configuration coupante), d'une courbure du biseau de l'embout vers l'ouverture de l'embout, etc.

On décrira ci-après diverses réalisations d'un embout tubulaire pour une aiguille utilisable en ponction veineuse et conforme à la présente invention, en référence aux figures du dessin joint sur lequel :
- la figure 1 représente un embout E dont le biseau coupant est de type « Lancet Point » c'est-à-dire un biseau simple réalisé par rectification selon un angle, cet embout étant vu de profil (fig.1-A) et de face (fig.1-B) ;
- la figure 2 représente l'embout de la figure 1 après une déformation, cet embout étant vu de profil (fig.2-A) et de face (fig.2-B) ;
- la figure 3 représente un embout E dont le biseau coupant est de type « Back Cut », c'est-à-dire un biseau lancet-point avec deux affûtages à l'arrière de la pointe, cet embout étant vu de profil (fig.3-A) et de face (fig.3-B) ;

- la figure 4 représente l'embout de la figure 3 après déformation, cet embout étant vu de profil (fig.4-A) et de face (fig.4-B) ;
- les figures 5 à 8 représentent de profil (figures A) et de face (figures B), divers cas de déformation d'un embout coupant E (Lancet Point ou Back Cut), conformément à l'invention ;
- la figure 9 est un schéma longitudinal d'un cathéter court connu enfilé sur une aiguille, avant usage ;
- la figure 10 est un schéma longitudinal du cathéter de la figure 9 ;
- la figure 11 est un schéma longitudinal de l'aiguille du cathéter de la figure 9 ;
- la figure 12 est un schéma longitudinal d'un cathéter court conforme à l'invention, avant usage, et
- la figure 13 est un schéma longitudinal du cathéter court de la figure 12 après déformation.

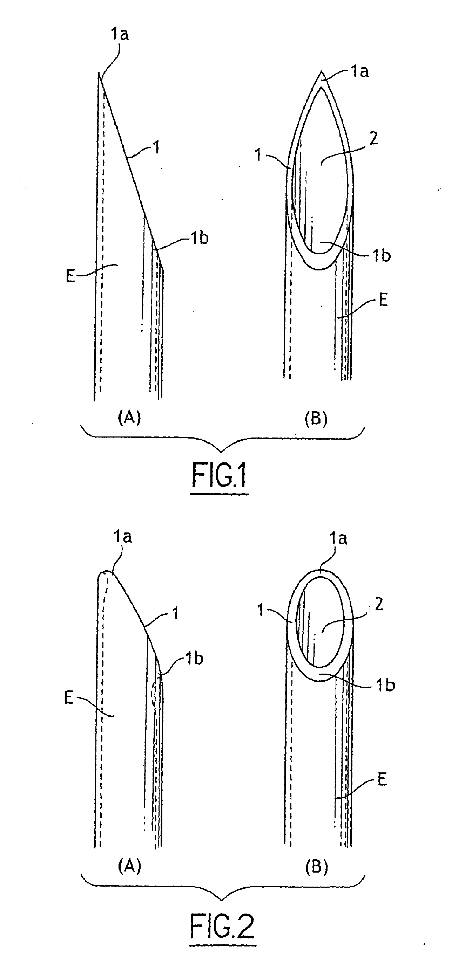

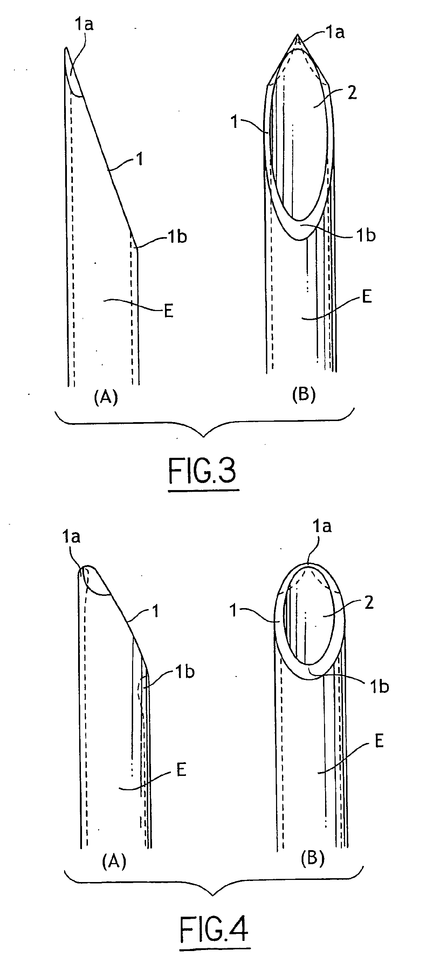

Une aiguille utilisée en ponction veineuse et artérielle a généralement un biseau coupant avec deux types de formes :
- 1^{er} type : Lancet point. C'est un biseau simple réalisé par rectification selon un angle. Sur la figure 1, on a mis un angle de 17°, il peut être différent selon les habitudes et les utilisations.
- 2^{ème} type: Back Cut (figure 3). C'est un biseau lancet-point avec deux affûtages à l'arrière de la pointe. Cela crée un amincissement de la pointe en même temps qu'un petit déplacement de celle-ci vers le centre du tube.

Une aiguille lancet-point au passage de la peau crée une trace en forme de croissant de lune. La back-cut crée un trou, ce qui est plus favorable au passage ultérieur d'une canule (exemple avec les cathéters courts).

Les déformations possibles de la forme du biseau pour le rendre atraumatique après insertion dans la veine, peuvent être :
- un émoussage de tous les bords du biseau coupant par arrondissement,
- un raccourcissement de la longueur du biseau coupant,
- un épaississement des bords du biseau coupant en plus de l'arrondi ;
- un arrondissement de la pointe du biseau coupant,
- un boulage de la pointe du biseau coupant du bout de l'aiguille avec les bords du biseau qui reviennent vers le centre ;
- un aplatissement partiel de la pointe du biseau coupant ;
- un courbage vers le centre du côté le plus long du biseau coupant (type aiguille Tuohy).

La déformation est rendue possible par un choix approprié d'un matériau à mémoire de forme pour constituer l'embout biseauté.

Comme cela a été rappelé par et dans les publications citées plus haut, la mise en oeuvre de la mémoire de forme pour obtenir une déformation avantageuse et contrôlée d'un tube est connue par l'homme du métier depuis de nombreuses années : le mode de déclenchement de cette déformation et la connaissance de la déformation recherchée permettent de choisir en conséquence le matériau et/ou la structure composite du tube à déformer.

A titre d'exemple, on a indiqué sur les figures 2 et 4 à 8 des déformations susceptibles de rendre atraumatique un embout biseauté coupant :
- figures 2 et 4 : la paroi du biseau (1) de l'embout E s'arrondit sur toute la circonférence de l'orifice (2) du biseau et la longueur du biseau se réduit. Ceci peut s'accompagner d'un épaississement de la paroi au bout ;

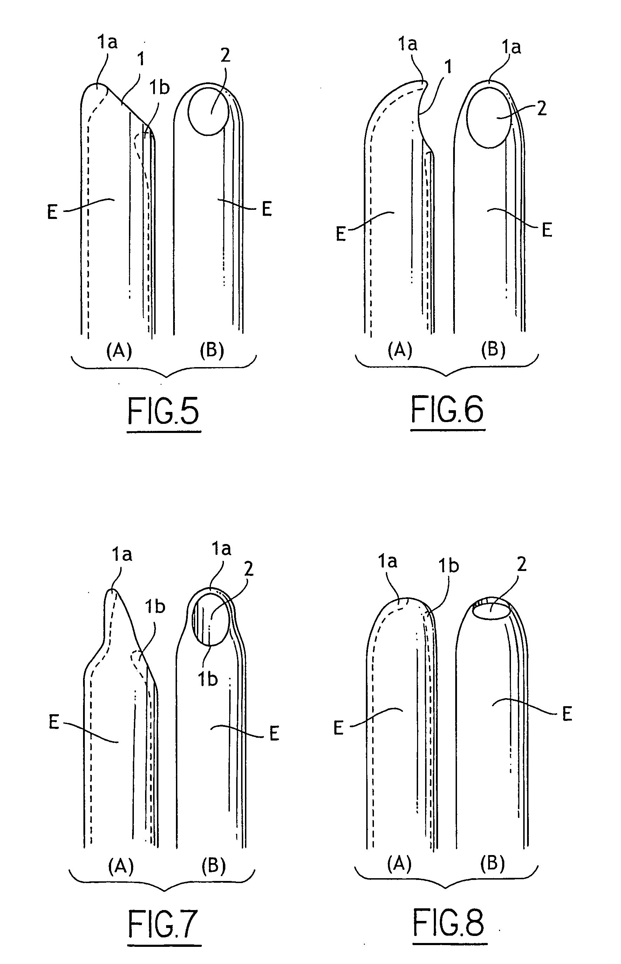

- figure 5 : le biseau de l'embout E s'affaisse à l'intérieur du tube ;
- figure 6 : l'extrémité haute (1a) du biseau de l'embout E se courbe vers l'intérieur et l'orifice (2) s' arrondit ;
- figure 7 : le biseau s'affaisse vers l'intérieur du tube et l'orifice (2) du tube s'arrondit ;
- figure 8 : les extrémités haute (1a) et basse (1b) du biseau (1) de l'embout E se rapprochent l'une de l'autre.

L'invention s'applique notamment aux aiguilles utilisées en cathétérisme veineux ou artériel ou blocs plexiques ou péridurale et sur tous les cathéters courts. Dans le cas du cathéter court, il peut être comme actuellement une aiguille + une canule mais dans une réalisation préférée l'aiguille devient en se ramollissant une canule atraumatique.

Un cathéter court est habituellement composé d'un tube en métal inoxydable qui présente à son extrémité distale un biseau coupant (biseautée le plus souvent selon un angle de 17°), et dont l'extrémité proximale est munie d'une embase surmoulée équipée d'un bouchon airvent laissant passer l'air, mais pas les liquides, donc pas le sang (filtre à membrane poreuse hydrophobe). C'est ce filtre qui permet en voyant le reflux sanguin dans le bouchon airvent transparent de savoir que le biseau du tube est bien dans la veine, et que l'on peut pousser la canule. Sur cette aiguille est montée une canule, qui doit être ajustée en diamètre, conique en bout (pour diminuer l'effort de pénétration dans la peau) et ajustée linéairement (généralement le bout de la canule doit être à moins d'un millimètre du bas du biseau du tube métallique).

Cette canule est généralement composée d'un tube en Téflon (PTFE) ou le plus souvent maintenant en polyuréthane (PUR) qui est thermosensible (il s'assouplit dans la veine et peut être toléré plus longtemps dans celle-ci), muni à une extrémité d'une embase équipée d'une cône femelle luer verrouillable, la canule est monté sur l'aiguille en butée et l'ensemble canule/aiguille peut être siliconé (pour améliorer la glisse).

L'utilisateur pique, attend le reflux sanguin, pousse la canule dans la veine et retire l'aiguille, il connecte l'embase de la canule à un prolongateur ou à une ligne de perfusion et fixe celle-ci sur la peau (pansement, ...) .

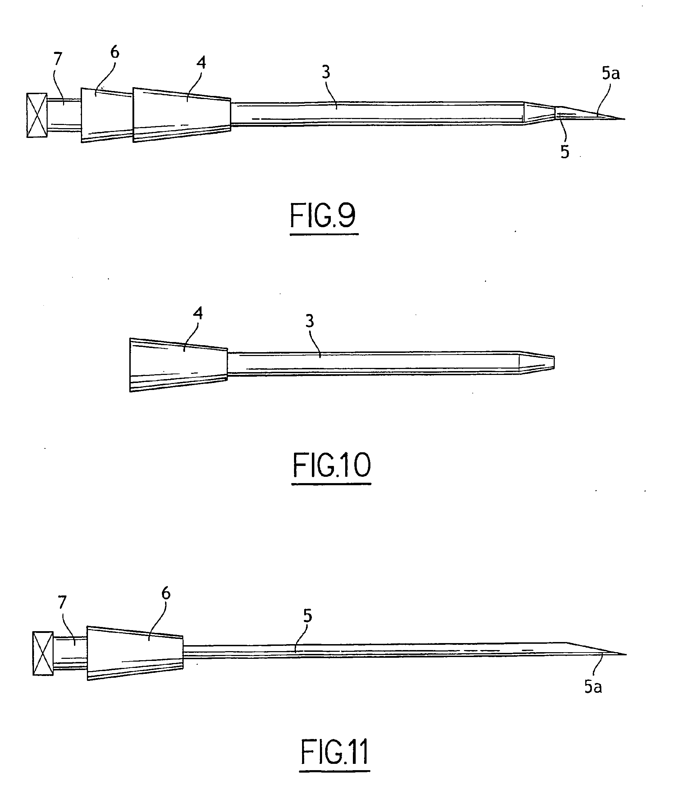

Pour l'exemple, on a représenté sur les figures 9 à 11 un cathéter court (3) en résine de synthèse avec son embase proximale (4), dans lequel est enfilée une aiguille métallique (5) à pointe biseautée (5a) et à embase proximale (6). Dans cet exemple, l'aiguille comporte en amont de son embase une chambre de visualisation du reflux sanguin avec son bouchon airvent (7).

Selon l'invention, un tel dispositif peut être sensiblement simplifié si l'aiguille et le cathéter peuvent se confondre. Le cathéter n'est alors composé que d'une canule, équipée d'un tube, d'une embase, d'un bouchon airvent monté directement sur cette embase. Le tube canule est en matériau plastique à mémoire de forme très rigide et a à son extrémité distale un biseau à 17° (ou à un angle différent) tranchant. Ce tube peut être siliconé.

Après piqûre et entrée du biseau dans la veine, le déclencheur (le sang par exemple) modifie la forme du biseau qui devient atraumatique (non tranchant, y compris dans la veine), on pousse la canule et le tube se ramollit au fur et à mesure de son introduction.

On enlève le bouchon airvent, on connecte le prolongateur ou la ligne de perfusion, et on fixe l'embase (avec un pansement, ou, ...).

On peut ressortir une canule souple et atraumatique. Avec un tel cathéter court, on économise l'aiguille, (ce qui est très important en terme de coût (coût de l'aiguille elle-même et coûts des ajustage de la canule sur l'aiguille), en économie de gestes (pas d'aiguille à enlever), et en diminution des risques de piqûres (maladies nosocomiales).

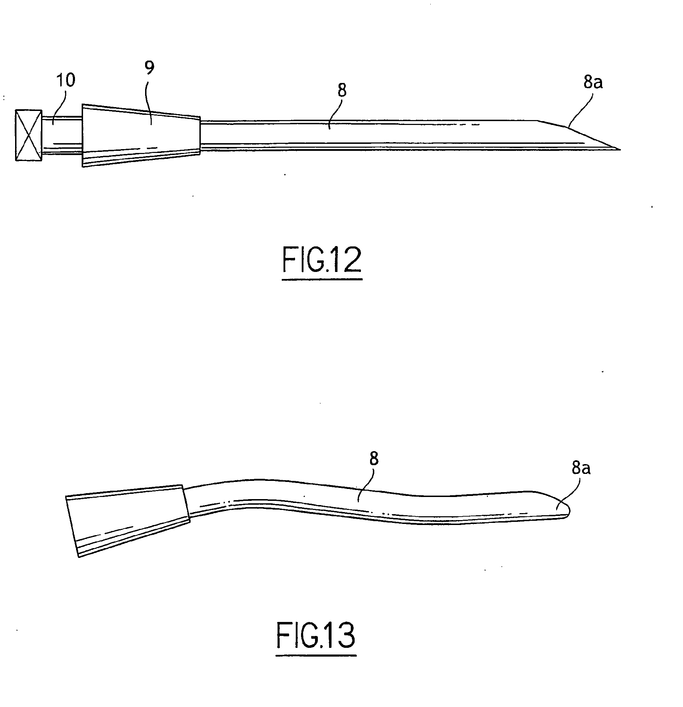

On utilise par exemple (fig.12) un tube en résine de synthèse à mémoire de forme (8) avec son embase (9) et une chambre de visualisation (10) rapportée de façon détachable sur l'embase du tube. Le tube est biseauté à son extrémité distale (8a) (ce biseau éventuellement précédé d'une pente douce) et est réalisé en matériau à mémoire de forme en sorte qu'avant déformation le tube soit suffisamment rigide pour servir d'aiguille en vue de la ponction et de l'introduction de sa pointe dans une veine et qu'après cette introduction, le tube présente une extrémité émoussée et une partie tubulaire ramollie et souple comme un cathéter classique.

L'invention peut être appliquée aux aiguilles simples dont le tube serait en matériau plastique rigide avec un biseau, ce matériau étant en mémoire de forme. On pique et l'extrémité tranchante dans la peau ou la veine ou l'artère devient atraumatique (trigger + liquide), on peut même canuler avec l'aiguille la veine ou l'artère (positionnement plus fiable).

Quand on retire l'aiguille, il n'y plus de risque de pique.

Il faut pour ce faire un matériau plastique très rigide et qui peut être tranchant avec un biseau moulé en forme.

Il faut que ce matériau soit très fluide lors de l'injection pour remplir des formes dans les mules très fines.

Il existe des matériaux plastiques déjà très rigides (PEEK : Polyéthyléthercétone : ou polyimide, ...), on peut imaginer qu'on arrivera encore à augmenter cette rigidité.

Le matériau plastique peut être aussi affûtable à la meule (comme le métal).

### EXEMPLE

Le matériau à mémoire de forme M est composé de deux matériaux :
- M1 qui se ramollit à température élevée et qui est plutôt mou ;
- M2 qui se ramollit à température basse et qui est très dur.
   - On pousse le tube M dans un moule à température élevée pour lui imposer la forme atraumatique choisie. Au refroidissement, cette forme est imposée à M1 et à M2.
   - On pousse le tube dans un deuxième moule à température basse, suffisante pour ramollir M2, mais pas M1. On peut rentrer aussi une tige dans le tube pour repousser des formes intérieures du premier moulage.

Au refroidissement M2 très dur impose la forme du deuxième moulage M1.

On peut affûter la forme pour la rendre plus coupante.

Quand on introduit l'ensemble de ponction dans la veine, soit la température de 37°C est suffisante pour ramollir M1, mais c'est quand même un peu bas (compte tenu de la température de stérilisation préalable (50°C), de l'affûtage qui peut l'échauffer, du stockage en été, ...), soit le matériau dur est très hydrophile et se ramollit très vite au contact de l'eau saline (comme les lentilles oculaires), permettant à M1 d'imposer sa forme, soit on rentre un mandrin chaud ou chauffable électriquement dans l'ensemble de ponction pour ramollir M2.

L'invention n'est pas limitée aux exemples de réalisation qui ont été décrits.

## Revendications

1. Embout de ponction E utilisable dans le domaine médical pour percer la peau et pénétrer dans un site du corps, cet embout étant constitué par un tronçon de tube creux en matériau à mémoire de forme qui présente à son extrémité distale un biseau coupant, stable et rigide, apte à découper la peau et pénétrer dans le site, **caractérisé en ce que** le biseau coupant a la mémoire d'un état où il est non coupant et **en ce qu'**il est apte à changer d'état pour reprendre cet état atraumatique lorsqu'il est soumis à une condition prévalente dans le site ou sous l'action d'un stimuli commandé de l'extérieur.

2. Embout selon la revendication 1 dont le matériau est choisi en sorte que le changement d'état du biseau soit déclenché par une élévation de température à l'intérieur du site.

3. Embout selon la revendication 1 dont le matériau est choisi en sorte que le changement d'état soit déclenché par un contact avec un liquide à l'intérieur du site.

4. Embout selon la revendication 1 dont le matériau est choisi en sorte que le changement d'état soit déclenché par une incitation électrique commandé de l'extérieur du site.

5. Embout selon l'une des revendications 1 à 4 dont le biseau non coupant a une forme qui diffère de la forme du biseau coupant par un ou plusieurs des changements suivants : émoussage de tous les bords du biseau coupant, raccourcissement de la longueur du biseau coupant, épaississement des bords du biseau coupant, arrondissement de la pointe du biseau coupant, boulage de la pointe du biseau coupant, aplatissement partiel de la pointe du biseau coupant, courbage du côté le plus long du biseau coupant vers le centre.

6. Embout selon l'une des revendications 1 à 5 composé de matériaux (M1, M2) aptes à être chauffés pour permettre de mouler l'embout à la forme atraumatique souhaitée et aptes à permettre de contraindre le biseau de l'embout à prendre la forme coupante, l'un (M2) de ces matériaux étant apte à maintenir le biseau de l'embout l'embout refroidi à la forme coupante tant que l'embout n'est pas soumis à ladite condition ou audit stimuli.

7. Embout selon l'une des revendications 1 à 6 réalisé en matériaux polymères.

8. Embout selon l'une des revendications 1 à 7 dont le biseau coupant (1) est du type « Lancet Point ».

9. Embout selon l'une des revendications 1 à 7 dont le biseau coupant (1) est du type « Back-cut ».

10. Embout selon l'une des revendications 1 à 9 et dans lequel le biseau coupant (1) est précédé d'une pente plus douce.

11. Embout selon l'une des revendications 1 à 10 et qui constitue une extrémité (8a) d'un tube (8).

12. Tube (8) muni d'un embout (8a) selon l'une des revendications 1 à 10 venu de fabrication avec le tube en un matériau à mémoire de forme pour pouvoir passer sous l'effet d'une condition existant dans le site d'un état où le tube a la rigidité requise pour servir à pousser l'embout jusque dans le site à un état où le tube est souple.

## Claims

1. A puncture tip E that is usable in the medical field to pierce the skin and penetrate into a site of the body, this needle-tip being composed of a section of hollow tube in a shape-memory material that, at its distal end, has a stable and rigid cutting bevel, designed to cut the skin and penetrate into the site, **characterized in that** the cutting bevel remembers a state in which it is not sharp, and **in that** it is capable of changing state so as to return to this atraumatic state when it is subjected to a condition that is prevalent at the site, or under the action of a stimulus controlled from the exterior.

2. The needle-tip according to claim 1, whose material is chosen so that the change of state of the bevel is triggered by a rise in temperature within the site.

3. The needle-tip according to claim 1, whose material is chosen so that the change of state is triggered by contact with a liquid within the site.

4. The needle-tip according to claim 1 whose material is chosen so that the change of state is triggered by an electrical stimulus controlled from the exterior of the site.

5. The needle-tip according to one of claims 1 to 4, whose bevel when not sharp has a shape which differs from the shape of the cutting bevel by one or more of the following changes: dulling of all the edges of the cutting bevel, shortening of the length of the cutting bevel, thickening of the edges of the cutting bevel, rounding of the point of the cutting bevel, balling of the point of the cutting bevel, partial flattening of the point of the cutting bevel, or curvature of the longest side of the cutting bevel toward the centre.

6. The needle-tip according to one of claims 1 to 5, composed of materials (M1, M2) designed to be heated to allow moulding of the needle-tip to the desired atraumatic shape, and designed to allow the bevel of the needle-tip to be stressed so that it assumes the cutting shape, one (M2) of these materials being capable of keeping the bevel of the cooled needle-tip in the cutting shape for as long as the needle-tip is not subjected to said condition or to the said stimulus.

7. The needle-tip according to one of claims 1 to 6, formed from polymer materials.

8. The needle-tip according to one of claims 1 to 7 whose cutting bevel (1) is of the "Lancet Point" type.

9. The needle-tip according to one of claims 1 to 7 whose cutting bevel (1) is of the "Back-cut" type.

10. The needle-tip according to one of claims 1 to 9, wherein the cutting bevel (1) is preceded by a gentler slope.

11. The needle-tip according to one of claims 1 to 10 and which constitutes one end (8a) of a tube (8).

12. A tube (8) fitted with a needle-tip (8a) according to one of claims 1 to 10, manufactured together with the tube in a shape-memory material so that, under the effect of a condition that exists at the site, it can change from a state in which the tube has the rigidity required so that the needle-tip can be pushed as far as the site, to a state in which the tube is flexible.

## Patentansprüche

1. Punktionsansatzstück E, welches im medizinischen Bereich verwendbar ist, um die Haut zu durchstechen und in eine Stelle des Körpers einzudringen, wobei das Ansatzstück aus einem hohlen Röhrenabschnitt aus Formgedächtnismaterial besteht, welcher an seinem distalen Ende eine stabile und starre schneidende Kante aufweist, welche die Haut zerschneiden und in die Stelle eindringen kann, **dadurch gekennzeichnet, dass** die schneidende Kante die Erinnerung an einen Zustand hat, in dem sie nicht schneidend ist, und dass sie, sobald sie einer an der Stelle vorherrschenden Bedingung unterworfen wird, oder unter Einwirkung eines von außen gesteuerten Reizes den Zustand ändern kann, um wieder den atraumatischen Zustand einzunehmen.

2. Ansatzstück gemäß Anspruch 1, wobei das Material derart ausgewählt ist, dass die Änderung des Zustands der Kante durch eine Erhöhung der Temperatur im Inneren der Stelle ausgelöst wird.

3. Ansatzstück gemäß Anspruch 1, wobei das Material derart ausgewählt ist, dass die Änderung des Zustands durch einen Kontakt mit einer Flüssigkeit im Inneren der Stelle ausgelöst wird.

4. Ansatzstück gemäß Anspruch 1, wobei das Material derart ausgewählt ist, dass die Änderung des Zustands durch einen elektrischen Reiz, der von außerhalb der Stelle gesteuert wird, ausgelöst wird.

5. Ansatzstück gemäß einem der Ansprüche 1 bis 4, wobei die nicht schneidende Kante eine Form aufweist, die sich von der Form der schneidenden Kante durch eine oder mehrere der folgenden Veränderungen unterscheidet: Abstumpfung sämtlicher Ränder der schneidenden Kante, Verkürzung der Länge der schneidenden Kante, Verdickung der Ränder der schneidenden kante, Abrundung der Spitze der schneidenden Kante, Kugelung der Spitze der schneidenden Kante, partielle Abplattung der Spitze der schneidenden Kante, Krümmen der längeren Seite der schneidenden Kante in Richtung Mitte.

6. Ansatzstück gemäß einem der Ansprüche 1 bis 5, bestehend aus Materialien (M1, M2), die erhitzt werden können, um ein Formen des Ansatzstücks in die gewünschte atraumatische Form zu ermöglichen, und die die Kante des Ansatzstücks dazu zwingen können, die schneidende Form anzunehmen, wobei eines (M2) dieser Materialien die Kante des abgekühlten Ansatzstücks in der scheidenden Form halten kann, solange das Ansatzstück nicht der besagten Bedingung oder dem besagten Reiz ausgesetzt wird.

7. Ansatzstück gemäß einem der Ansprüche 1 bis 6, welches aus Polymermaterialien hergestellt ist.

8. Ansatzstück gemäß einem der Ansprüche 1 bis 7, wobei die schneidende Kante (1) vom "Lancet-Point"-Typ ist.

9. Ansatzstück gemäß einem der Ansprüche 1 bis 7, wobei die schneidende Kante (1) vom "Back-cut"-Typ ist.

10. Ansatzstück gemäß einem der Ansprüche 1 bis 9, wobei die schneidende Kante (1) mit einer sanfteren Schräge versehen ist.

11. Ansatzstück gemäß einem der Ansprüche 1 bis 10, welches ein Ende (8a) einer Röhre (8) bildet.

12. Röhre (8), welche mit einem Ansatzstück (8a) gemäß einem der Ansprüche 1 bis 10 versehen ist und mit der Röhre aus einem Formgedächtnismaterial hergestellt ist, um unter der Wirkung einer an der Stelle existierenden Bedingung von einem Zustand, in dem die Röhre die erforderliche Steifheit besitzt, um das Ansatzstück bis zu der Stelle vorwärts zu treiben, in einen Zustand, in dem die Röhre biegsam ist, übergehen zu können.
